# FASCICULE DE BREVET EUROPEEN

(11) **EP 3 641 920 B1**
(45) Date de publication et mention de la délivrance du brevet: **05.01.2022**
(21) Numéro de dépôt: 18752527.4
(22) Date de dépôt: 12.06.2018
(51) Int. Cl.: B01F 3/20, B01F 11/00, B01F 15/02

(54) **APPAREIL DE FABRICATION D'UN PRODUIT COSMÉTIQUE**
VORRICHTUNG ZUR HERSTELLUNG EINES KOSMETIKUMS
APPARATUS FOR PRODUCING A COSMETIC

(30) Priorité: 23.06.2017 FR 1755744
(43) Date de publication de la demande: 29.04.2020
(73) Titulaire: SEB S.A., 69130 Ecully (FR); Duolab International Sàrl, 1228 Plan-Les-Ouates (CH)
(72) Inventeur: LE GRAND, Olivier, 21380 Messigny Et Vantoux (FR); DE BRUGIERE, Philippe, 78160 Marly Le Roi (FR)
(74) Mandataire: Germain Maureau
(86) Numéro de dépôt international: PCT/FR2018/051379
(87) Numéro de publication internationale: WO 2018/234658

(56) Documents cités:
- DE-U1- 9 017 592
- FR-A1- 3 026 622

## Description

La présente invention concerne un appareil de fabrication d'un produit cosmétique.

Le document FR3026622 divulgue un appareil selon le préambule de la revendication 1. Il divulgue un appareil de fabrication d'un produit cosmétique, comportant :
- une première capsule contenant une quantité prédéterminée d'une première formulation, la première capsule comportant une première partie de connexion et un passage de sortie pourvu d'un orifice de sortie,
- une deuxième capsule contenant une quantité prédéterminée d'une deuxième formulation, et comportant une deuxième partie de connexion configurée pour être connectée à la première partie de connexion, et
- une machine de mélange configurée pour :
- recevoir les première et deuxième capsules,
- obturer le passage de sortie,
- mélanger les première et deuxième formulations à l'intérieur des première et deuxième capsules de manière à obtenir le produit cosmétique, et
- libérer le passage de sortie de manière à permettre ensuite l'évacuation du produit cosmétique fabriqué hors de la première capsule et la récupération du produit cosmétique fabriqué sur une coupelle de réception.

En particulier, une telle machine de mélange comporte un élément d'obturation déplaçable entre une position d'obturation du passage de sortie et une position de libération du passage de sortie, et une unité de commande configurée pour commander les déplacements de l'élément d'obturation entre les positions d'obturation et de libération.

Un tel appareil de fabrication permet la fabrication, par un consommateur final, d'un produit cosmétique personnalisé à partir de différentes capsules.

Cependant, la structure de l'appareil de fabrication décrit dans le document FR3026622 pourrait encore être simplifiée. De plus, l'utilisation de l'appareil de fabrication décrit dans le document FR3026622 peut s'avérer complexe pour certains consommateurs, notamment lorsqu'il est nécessaire de réaliser une connexion des première et deuxième capsules préalablement à leur insertion dans la machine de mélange.

La présente invention vise à remédier à tout ou partie de ces inconvénients.

Le problème technique à la base de l'invention consiste donc à fournir un appareil de fabrication d'un produit cosmétique qui soit simple et facile d'utilisation, tout en ayant une structure simple et fiable.

A cet effet, la présente invention concerne un appareil de fabrication d'un produit cosmétique, comprenant :
- une première capsule contenant une quantité prédéterminée d'une première formulation, la première capsule comportant une première partie de connexion et un passage de sortie pourvu d'un orifice de sortie,
- une deuxième capsule contenant une quantité prédéterminée d'une deuxième formulation, et comportant une deuxième partie de connexion configurée pour être connectée à la première partie de connexion,
- une machine de mélange configurée pour recevoir les première et deuxième capsules, de préférence reliées fluidiquement l'une à l'autre par les première et deuxième parties de connexion, et pour mélanger les première et deuxième formulations contenues dans les première et deuxième capsules de manière à obtenir le produit cosmétique, le passage de sortie étant relié fluidiquement à la première partie de connexion, caractérisé en ce que l'appareil de fabrication est configuré pour automatiquement obturer le passage de sortie lorsque les première et deuxième capsules sont reçues dans la machine de mélange, et pour automatiquement libérer le passage de sortie lorsque les première et deuxième capsules sont retirées hors de la machine de mélange.

Une telle configuration de l'appareil de fabrication, et notamment l'obturation automatique du passage de sortie lors de la réception des première et deuxième capsules dans la machine de mélange, assure une obturation simple et fiable du passage de sortie, et notamment pendant le mélange des première et deuxième formulations.

De plus, la libération automatique du passage de sortie, lors du retrait des première et deuxième capsules hors de la machine de mélange, assure une libération simple et fiable du passage de sortie, sans nécessiter la commande d'un actionneur particulier.

Ainsi, l'appareil de fabrication selon la présente invention présente une structure simple et fiable, tout en étant simple et facile d'utilisation.

Dans la présente invention, on entend par « relier fluidiquement », le fait qu'une communication fluidique peut être établie entre les différents éléments associés au terme « relier fluidiquement ».

L'appareil de fabrication peut en outre présenter une ou plusieurs des caractéristiques suivantes, prises seules ou en combinaison.

Selon un mode de réalisation de l'invention, l'appareil de fabrication est configuré pour automatiquement obturer le passage de sortie concomitamment à l'insertion des première et deuxième capsules dans la machine de mélange, et pour automatiquement libérer le passage de sortie concomitamment au retrait des première et deuxième capsules hors de la machine de mélange

Selon un mode de réalisation de l'invention, l'appareil de fabrication comporte en outre un dispositif de réception configuré pour recevoir les première et deuxième capsules, la machine de mélange étant configurée pour recevoir le dispositif de réception équipé des première et deuxième capsules.

Selon un mode de réalisation de l'invention, le dispositif de réception est apte à occuper une position ouverte dans laquelle les première et deuxième capsules sont aptes à être introduites dans le dispositif de réception, et une position fermée dans laquelle le dispositif de réception est apte à maintenir en position les première et deuxième capsules.

Selon un mode de réalisation de l'invention, le dispositif de réception est configuré pour relier fluidiquement les première et deuxième parties de connexion lorsque le dispositif de réception est déplacé dans la position fermée.

Selon un mode de réalisation de l'invention, le dispositif de réception comporte un boîtier de réception configuré pour recevoir et loger au moins en partie les première et deuxième capsules.

Avantageusement, l'appareil de fabrication est configuré pour automatiquement obturer le passage de sortie lorsque le dispositif de réception équipé des première et deuxième capsules est reçu dans la machine de mélange, et pour automatiquement libérer le passage de sortie lorsque le dispositif de réception est retiré hors de la machine de mélange.

Selon un mode de réalisation de l'invention, la première capsule comporte un premier compartiment déformable contenant la première formulation, et la deuxième capsule comporte un deuxième compartiment déformable contenant la deuxième formulation.

Selon un mode de réalisation de l'invention, la première capsule comporte en outre un premier passage de liaison configuré pour relier fluidiquement le premier compartiment déformable et la première partie de connexion, et la deuxième capsule comporte en outre un deuxième passage de liaison configuré pour relier fluidiquement le deuxième compartiment déformable et la deuxième partie de connexion.

Selon un mode de réalisation de l'invention, le passage de sortie est relié fluidiquement au premier passage de liaison. Par exemple, le passage de sortie peut déboucher dans le premier passage de liaison

Selon un mode de réalisation de l'invention, le passage de sortie s'étend dans le prolongement du premier passage de liaison.

Avantageusement, le passage de sortie s'étend sensiblement parallèlement au premier passage de liaison.

Selon un mode de réalisation de l'invention, la première partie de connexion s'étend sensiblement perpendiculairement par rapport au premier passage de liaison.

Selon un mode de réalisation de l'invention, l'appareil de fabrication comporte un élément d'obturation configuré pour obturer automatiquement le passage de sortie lorsque les première et deuxième capsules sont reçues dans la machine de mélange, par exemple lorsque le dispositif de réception équipé des première et deuxième capsules est reçu dans la machine de mélange, et pour libérer automatiquement le passage de sortie lorsque les première et deuxième capsules sont retirées hors de la machine de mélange, et par exemple lorsque le dispositif de réception équipé des première et deuxième capsules est retiré hors de la machine de mélange.

Selon un mode de réalisation de l'invention, l'élément d'obturation est configuré pour pincer le passage de sortie ou pour exercer une pression sur le passage de sortie.

Selon un mode de réalisation de l'invention, l'élément d'obturation est monté mobile entre une position d'obturation dans laquelle l'élément d'obturation est apte à obturer le passage de sortie, et par exemple apte à pincer le passage de sortie ou exercer une pression sur le passage de sortie, et une position de libération dans laquelle l'élément d'obturation est apte à libérer le passage de sortie.

Selon un mode de réalisation de l'invention, la machine de mélange comporte un élément de déplacement configuré pour déplacer l'élément d'obturation dans la position d'obturation lorsque les première et deuxième capsules sont reçues dans la machine de mélange, et par exemple lorsque le dispositif de réception est inséré dans la machine de mélange.

Selon un mode de réalisation de l'invention, l'appareil de fabrication est configuré de telle sorte que l'introduction du dispositif de réception dans la machine de mélange jusqu'à une position de butée du dispositif de réception entraîne mécaniquement un déplacement de l'élément d'obturation dans la position d'obturation. Ces dispositions permettent notamment d'éviter la présence d'un détecteur de fin de course, et d'un actionneur couplé au détecteur de fin de course et apte à déplacer l'élément d'obturation dans la position d'obturation.

Selon un mode de réalisation de l'invention, l'appareil de fabrication est configuré de telle sorte que le retrait du dispositif de réception hors de la machine de mélange entraîne mécaniquement un déplacement de l'élément d'obturation dans la position de libération.

Selon un mode de réalisation de l'invention, l'appareil de fabrication comporte un élément de contre-appui configuré pour prendre appui contre la première capsule et pour être disposé en regard de l'élément d'obturation lorsque les première et deuxième capsules sont reçues dans la machine de mélange, et par exemple lorsque le dispositif de réception équipé des première et deuxième capsules est reçu dans la machine de mélange.

Avantageusement, l'élément de contre-appui est configuré pour être disposé en regard de l'élément d'obturation lorsque le dispositif de réception est dans la position fermée.

Selon un mode de réalisation de l'invention, le dispositif de réception comporte l'élément de contre-appui.

Selon un mode de réalisation de l'invention, le premier passage de liaison est un premier canal de liaison, et le deuxième passage de liaison est un deuxième canal de liaison.

Selon un mode de réalisation de l'invention, le passage de sortie est un canal de sortie.

Selon un mode de réalisation de l'invention, chacune des première et deuxième capsules est à usage unique.

Selon un mode de réalisation de l'invention, le dispositif de réception comporte l'élément d'obturation.

Selon un mode de réalisation de l'invention, la première capsule comporte une coque thermoformée et une feuille d'obturation recouvrant la coque thermoformée, la coque thermoformée et la feuille d'obturation de la première capsule délimitant le passage de sortie.

Selon un mode de réalisation de l'invention, la deuxième capsule comporte également une coque thermoformée et une feuille d'obturation recouvrant la coque thermoformée respective, la coque thermoformée et la feuille d'obturation de la première capsule délimitant le premier compartiment déformable et le premier passage de liaison, et la coque thermoformée et la feuille d'obturation de la deuxième capsule délimitant le deuxième compartiment déformable et le deuxième passage de liaison.

Selon un mode de réalisation de l'invention, la machine de mélange est configurée pour mélanger les première et deuxième formulations à l'intérieur des première et deuxième capsules. Une telle configuration permet au moins d'éviter un contact entre la machine de mélange et les première et deuxième formulations, et donc d'éviter un nettoyage ultérieur de la machine de mélange après la fabrication du produit cosmétique.

Selon un mode de réalisation de l'invention, l'appareil de fabrication comporte un élément de chauffage configuré pour chauffer au moins l'une des première et deuxième capsules lorsque les première et deuxième capsules sont reçues dans la machine de mélange, et par exemple le dispositif de réception équipé des première et deuxième capsules est reçu dans la machine de mélange.

Selon un mode de réalisation de l'invention, l'élément de chauffage est configuré pour chauffer la première capsule. Avantageusement, l'élément de chauffage est configuré pour s'étendre le long d'une surface extérieure de la première capsule.

Selon un mode de réalisation de l'invention, l'élément de chauffage est disposé dans le dispositif de réception ou dans la machine de mélange.

Selon un mode de réalisation de l'invention, l'élément de chauffage est configuré pour s'étendre entre les première et deuxième capsules.

Selon un mode de réalisation de l'invention, l'élément de chauffage est disposé dans la machine de mélange et est configuré pour s'étendre entre les première et deuxième capsules lorsque le dispositif de réception équipé des première et deuxième capsules est reçu dans la machine de mélange.

Selon un autre mode de réalisation de l'invention, l'élément de chauffage est disposé dans le dispositif de réception et est configuré pour s'étendre entre les première et deuxième capsules lorsque les première et deuxième capsules sont reçues dans le dispositif de réception.

Selon un mode de réalisation de l'invention, l'appareil de fabrication comporte un premier organe d'actionnement configuré pour transmettre un effort de pression à la première capsule, et un deuxième organe d'actionnement configuré pour transmettre un effort de pression à la deuxième capsule. Par exemple, la machine de mélange comporte les premier et deuxième organes d'actionnement.

Selon un autre mode de réalisation de l'invention, le premier organe d'actionnement est configuré pour transmettre un effort de pression à la première capsule de manière à faire migrer le contenu de la première capsule dans la deuxième capsule, et le deuxième organe d'actionnement est configuré pour transmettre un effort de pression à la deuxième capsule de manière à faire migrer le contenu de la deuxième capsule dans la première capsule.

Selon un mode de réalisation de l'invention, les premier et deuxième organes d'actionnement sont configurés pour transmettre alternativement des efforts de pression respectivement aux première et deuxième capsules.

Selon un mode de réalisation de l'invention, les premier et deuxième organes d'actionnement sont disposés en regard l'un de l'autre.

Selon un autre mode de réalisation de l'invention, les premier et deuxième organes d'actionnement sont configurés pour être disposés de part et d'autre du dispositif de réception lorsque le dispositif de réception est reçu dans la machine de mélange.

Selon un autre mode de réalisation de l'invention, la machine de mélange comporte :
- une partie d'actionnement comportant les premier et deuxième organes d'actionnement, la partie d'actionnement étant montée pivotante autour d'un axe de pivotement, et
- un moteur d'entraînement configuré pour faire pivoter la partie d'actionnement autour de l'axe de pivotement et alternativement dans un premier sens de pivotement et dans un deuxième sens de pivotement opposé au premier sens de pivotement.

Selon un mode de réalisation de l'invention, le moteur d'entraînement est configuré pour faire pivoter la partie d'actionnement autour de l'axe de pivotement et dans une plage angulaire prédéterminée.

Selon un mode de réalisation de l'invention, les premier et deuxième organes d'actionnement convergent à l'opposé de l'axe de pivotement.

Selon un mode de réalisation de l'invention, la machine de mélange est configurée de telle sorte qu'une rotation du moteur d'entraînement dans un premier sens de rotation entraîne un pivotement de la partie d'actionnement dans le premier sens de pivotement et qu'une rotation du moteur d'entraînement dans un deuxième sens de rotation, opposé au premier sens de rotation, entraîne un pivotement de la partie d'actionnement dans le deuxième sens de pivotement.

Selon un autre mode de réalisation de l'invention, la machine de mélange comporte en outre une roue d'entraînement solidaire en rotation d'un arbre de sortie du moteur d'entraînement et configurée pour être entraînée en rotation autour de son axe de roue par le moteur d'entraînement, la roue d'entraînement étant équipée d'un élément d'entraînement, tel qu'un doigt d'entraînement, excentré par rapport à l'axe de roue, et la partie d'actionnement comporte une lumière de réception qui est allongée et dans laquelle est reçu l'élément d'entraînement.

Selon un mode de réalisation de l'invention, la lumière de réception s'étend selon une direction d'extension sensiblement parallèle à l'axe de pivotement.

Selon un mode de réalisation de l'invention, le dispositif de réception est configuré de telle sorte que, lorsque le dispositif de réception équipé des première et deuxième capsules est dans la position fermée, une projection orthogonale de la première capsule sur un plan de référence est au moins en partie confondue avec une projection orthogonale de la deuxième capsule sur le plan de référence. De façon avantageuse, le dispositif de réception est configuré de telle sorte que, lorsque le dispositif de réception équipé des première et deuxième capsules est dans la position fermée, une projection orthogonale du premier compartiment déformable de la première capsule sur un plan de référence est au moins en partie confondue avec une projection orthogonale du deuxième compartiment déformable de la deuxième capsule sur le plan de référence.

Selon un mode de réalisation de l'invention, le dispositif de réception est configuré de telle sorte que les première et deuxième capsules s'étendent sensiblement parallèlement l'une par rapport à l'autre lorsque le dispositif de réception équipé des première et deuxième capsules est dans la position fermée.

Selon un mode de réalisation de l'invention, le dispositif de réception comporte un élément de verrouillage configuré pour verrouiller le dispositif de réception dans la position fermée.

Selon un mode de réalisation de l'invention, l'élément de verrouillage est mobile entre une position de verrouillage dans laquelle l'élément de verrouillage verrouille le dispositif de réception dans la position fermée, et une position de déverrouillage dans laquelle l'élément de verrouillage autorise un déplacement du dispositif de réception dans la position ouverte.

Selon un mode de réalisation de l'invention, le dispositif de réception comporte un élément de libération, tel qu'un bouton de libération, configuré pour déplacer l'élément de verrouillage dans la position de déverrouillage.

Selon un mode de réalisation de l'invention, la première partie de connexion comporte un embout de connexion mâle, par exemple cylindrique, et la deuxième partie de connexion comporte un embout de connexion femelle, par exemple cylindrique, configuré pour recevoir l'embout de connexion mâle.

Selon un mode de réalisation de l'invention, le dispositif de réception comporte une première coque de protection et une deuxième coque de protection montées mobiles l'une par rapport à l'autre entre une première position correspondant à une position ouverte du dispositif de réception et une deuxième position correspondant à une position fermée du dispositif de réception.

Par exemple, les première et deuxième coques de protection sont montées articulées l'une par rapport à l'autre autour d'un axe d'articulation.

Avantageusement, l'élément d'obturation est monté mobile sur la première coque de protection.

Avantageusement, l'élément de contre-appui est prévu sur la deuxième coque de protection.

Selon un mode de réalisation de l'invention, le dispositif de réception comporte un premier emplacement de réception configuré pour recevoir la première capsule et un deuxième emplacement de réception configuré pour recevoir la deuxième capsule.

Selon un mode de réalisation de l'invention, le dispositif de réception comporte une première partie de support comportant le premier emplacement de réception et une deuxième partie de support comportant le deuxième emplacement de réception, les première et deuxième parties de support étant mobiles l'une par rapport à l'autre entre une position de réception dans laquelle les première et deuxième parties de support sont éloignées l'une de l'autre et les première et deuxième capsules sont aptes à être reçues respectivement dans les premier et deuxième emplacements de réception, et une position de connexion dans laquelle les première et deuxième parties de support sont rapprochées l'une de l'autre et les première et deuxième capsules sont aptes à être connectées l'une à l'autre.

Selon un mode de réalisation de l'invention, les première et deuxième parties de support sont configurées pour être déplacées dans la position de connexion lorsque le dispositif de réception est déplacé dans la position fermée.

Selon un mode de réalisation de l'invention, les première et deuxième parties de support sont montées articulées l'une par rapport à l'autre autour d'un axe d'articulation.

Selon un mode de réalisation de l'invention, les première et deuxième coques de protection et les première et deuxième parties de support sont montées articulées autour d'un même axe d'articulation.

Selon un mode de réalisation de l'invention, le premier emplacement de réception comporte une première rainure de réception configurée pour recevoir au moins une portion de la première capsule, et le deuxième emplacement de réception comporte une deuxième rainure de réception configurée pour recevoir au moins une portion de la deuxième capsule. Avantageusement, chacune des première et deuxième rainures de réception est sensiblement arquée. De façon avantageuse, chacune des première et deuxième rainures de réception est configurée pour coopérer avec un bord périphérique de l'une respective des première et deuxième capsules.

Selon un mode de réalisation de l'invention, la première partie de support comporte un premier élément de détrompage, tel qu'une première encoche de détrompage, configuré pour coopérer avec la première partie de connexion, et la deuxième partie de support comporte un deuxième élément de détrompage, tel qu'une deuxième encoche de détrompage, configuré pour coopérer avec la deuxième partie de connexion.

Selon un mode de réalisation de l'invention, la machine de mélange comporte un logement de réception configuré pour recevoir les première et deuxième capsules, et par exemple pour recevoir au moins en partie le dispositif de réception équipé des première et deuxième capsules.

Selon un mode de réalisation de l'invention, la machine de mélange comporte une ouverture d'insertion débouchant dans le logement de réception, les première et deuxième capsules étant configurées pour être insérées dans le logement de réception à travers l'ouverture d'insertion. Avantageusement, le dispositif de réception, équipé des première et deuxième capsules, est configuré pour être inséré dans le logement de réception à travers l'ouverture d'insertion. De façon avantageuse, l'ouverture d'insertion est configurée pour être orientée vers le haut lorsque la machine de mélange est disposée sur un support plan.

Selon un mode de réalisation de l'invention, la machine de mélange et le dispositif de réception sont configurés de telle sorte que le dispositif de réception s'étend au moins en partie à l'extérieur de la machine de mélange lorsque le dispositif de réception est reçu dans le logement de réception.

Selon un mode de réalisation de l'invention, la machine de mélange comporte une base présentant une surface supérieure sur laquelle est ménagée l'ouverture d'insertion.

Selon un mode de réalisation de l'invention, le logement de réception est situé dans une zone centrale de la base.

Selon un mode de réalisation de l'invention, la machine de mélange comporte une source d'alimentation électrique configurée pour alimenter électriquement la machine de mélange. Avantageusement, la source d'alimentation électrique comporte au moins une batterie rechargeable.

Selon un mode de réalisation de l'invention, la source d'alimentation électrique est configurée pour alimenter électriquement le dispositif de réception lorsque le dispositif de réception est reçu dans la machine de mélange.

Selon un mode de réalisation de l'invention, la machine de mélange comporte un premier connecteur électrique, et le dispositif de réception comporte un deuxième connecteur électrique configuré pour être connecté au premier connecteur électrique lorsque le dispositif de réception est reçu dans la machine de mélange, de telle sorte que la machine de mélange est apte à alimenter électriquement le dispositif de réception.

Selon un mode de réalisation de l'invention, l'appareil de fabrication comprend un contrôleur configuré pour contrôler le moteur d'entraînement et/ou l'élément de chauffage.

Selon un mode de réalisation de l'invention, l'élément de chauffage est configuré pour chauffer le premier compartiment déformable. Avantageusement, l'élément de chauffage est configuré pour s'étendre à proximité du premier compartiment déformable, et de préférence de manière adjacente au premier compartiment déformable.

Selon un mode de réalisation de l'invention, chacune des première et deuxième capsules est configurée pour contenir la totalité ou sensiblement la totalité d'un mélange formé par la quantité prédéterminée de la première formulation et la quantité prédéterminée de la deuxième formulation.

Selon un mode de réalisation de l'invention, la première formulation est une première phase du produit cosmétique, et la deuxième formulation est une deuxième phase du produit cosmétique.

Selon un mode de réalisation de l'invention, la première formulation est une phase grasse du produit cosmétique, et la deuxième formulation est une phase aqueuse du produit cosmétique. Par exemple, la phase grasse constitue la base du produit cosmétique à fabriquer, et la phase aqueuse comprend des éléments actifs et constitue le complexe d'actifs du produit cosmétique à fabriquer.

Selon un mode de réalisation de l'invention, le produit cosmétique fabriqué est une émulsion homogénéisée, une solution homogénéisée ou un mélange de plusieurs phases miscibles.

Selon un mode de réalisation de l'invention, la première capsule est configurée pour s'étendre au moins en partie à l'extérieur du dispositif de réception lorsqu'elle est reçue dans le dispositif de réception. Avantageusement, l'orifice de sortie est configuré pour s'étendre à l'extérieur du dispositif de réception lorsque la première capsule est reçue dans le dispositif de réception.

Selon un mode de réalisation de l'invention, le dispositif de réception comporte un premier élément d'appui configuré pour exercer un effort de pression sur la première capsule, et par exemple sur le premier compartiment déformable, lorsque le dispositif de réception est équipé des première et deuxième capsules, et un deuxième élément d'appui configuré pour exercer un effort de pression sur la deuxième capsule, et par exemple sur le deuxième compartiment déformable, lorsque le dispositif de réception est équipé des première et deuxième capsules.

Selon un mode de réalisation de l'invention, le premier élément d'appui s'étend à travers une première ouverture de passage prévue sur la première coque de protection, et le deuxième élément d'appui s'étend à travers une deuxième ouverture de passage prévue sur la deuxième coque de protection.

Selon un mode de réalisation de l'invention, le premier organe d'actionnement est configuré pour transmettre un effort de pression au premier élément d'appui, et le deuxième organe d'actionnement est configuré pour transmettre un effort de pression au deuxième élément d'appui.

Selon un mode de réalisation de l'invention, le premier élément d'appui est mobile et/ou déformable entre une position inactive et une position active dans laquelle le premier élément d'appui est apte à exercer un effort de pression sur la première capsule, et le deuxième élément d'appui est mobile et/ou déformable entre une position inactive et une position active dans laquelle le deuxième élément d'appui est apte à exercer un effort de pression sur la deuxième capsule. Chacun des premier et deuxième éléments d'appui est par exemple monté mobile en translation, par exemple selon une direction de déplacement qui est transversale, et de préférence sensiblement orthogonale, aux plans d'extension des première et deuxième capsules lorsque les première et deuxième capsules sont reçues dans la machine de mélange.

Avantageusement, la position inactive du premier élément d'appui correspond à une position du premier élément d'appui dans laquelle, lorsque le dispositif de réception est équipé des première et deuxième capsules :
- le premier élément d'appui est situé à distance de la première capsule, et plus particulièrement du premier compartiment déformable, ou
- le premier élément d'appui est uniquement au contact de la première capsule, et plus particulièrement du premier compartiment déformable, ou
- le premier élément d'appui exerce un effort de pression sur la première capsule, et plus particulièrement sur le premier compartiment déformable, qui est insuffisant pour faire migrer le contenu du premier compartiment déformable vers la deuxième capsule.

Avantageusement, la position inactive du deuxième élément d'appui correspond à une position du deuxième élément d'appui dans laquelle, lorsque le dispositif de réception est équipé des première et deuxième capsules :
- le deuxième élément d'appui est situé à distance de la deuxième capsule, et plus particulièrement du deuxième compartiment déformable, ou
- le deuxième élément d'appui est uniquement au contact de la deuxième capsule, et plus particulièrement du deuxième compartiment déformable, ou
- le deuxième élément d'appui exerce un effort de pression sur la deuxième capsule, et plus particulièrement sur le deuxième compartiment déformable, qui est insuffisant pour faire migrer le contenu du deuxième compartiment déformable vers la première capsule.

Avantageusement, la position active du premier élément d'appui correspond à une position du premier élément d'appui dans laquelle, lorsque le dispositif de réception est équipé des première et deuxième capsules, le premier élément d'appui exerce un effort de pression sur la première capsule, et plus particulièrement sur le premier compartiment déformable, qui est suffisant pour faire migrer le contenu du premier compartiment déformable vers la deuxième capsule, et la position active du deuxième élément d'appui correspond à une position du deuxième élément d'appui dans laquelle, lorsque le dispositif de réception est équipé des première et deuxième capsules, le deuxième élément d'appui exerce un effort de pression sur la deuxième capsule, et plus particulièrement sur le deuxième compartiment déformable, qui est suffisant pour faire migrer le contenu du deuxième compartiment déformable vers la première capsule.

Selon un mode de réalisation de l'invention, les premier et deuxième éléments d'appui sont montés respectivement sur les première et deuxième coques de protection.

Selon un mode de réalisation de l'invention, le premier élément d'appui comporte une première surface d'appui configurée pour coopérer avec la première capsule, et par exemple avec le premier compartiment déformable, la première surface d'appui étant configurée pour guider et orienter le contenu de la première capsule vers la première partie de connexion lorsque le premier élément d'appui exerce un effort de pression sur la première capsule, et le deuxième élément d'appui comporte une deuxième surface d'appui configurée pour coopérer avec la deuxième capsule, et par exemple avec le deuxième compartiment déformable, la deuxième surface d'appui étant configurée pour guider et orienter le contenu de la deuxième capsule vers la deuxième partie de connexion lorsque le deuxième élément d'appui exerce un effort de pression sur la deuxième capsule.

Selon un mode de réalisation de l'invention, lorsque le premier élément d'appui est déplacé de la position inactive vers la position active, la première surface d'appui est configurée pour exercer un effort d'appui successivement sur une première portion du premier compartiment déformable située à une première distance primaire du premier passage de liaison et sur une deuxième portion du premier compartiment déformable située à une deuxième distance primaire du premier passage de liaison, la deuxième distance primaire étant inférieure à la première distance primaire, et dans lequel, lorsque le deuxième élément d'appui est déplacé de la position inactive vers la position active, la deuxième surface d'appui est configurée pour exercer un effort d'appui successivement sur une première portion du deuxième compartiment déformable située à une première distance secondaire du deuxième passage de liaison et sur une deuxième portion du deuxième compartiment déformable située à une deuxième distance secondaire du deuxième passage de liaison, la deuxième distance secondaire étant inférieure à la première distance secondaire.

Selon un mode de réalisation de l'invention, la première surface d'appui comporte une première portion de surface primaire configurée pour exercer un effort de pression sur la première portion du premier compartiment déformable, et une deuxième portion de surface primaire configurée pour exercer un effort de pression sur la deuxième portion du premier compartiment déformable, et la deuxième surface d'appui comporte une première portion de surface secondaire configurée pour exercer un effort de pression sur la première portion du deuxième compartiment déformable, et une deuxième portion de surface secondaire configurée pour exercer un effort de pression sur la deuxième portion du deuxième compartiment déformable.

Selon un mode de réalisation de l'invention, les première et deuxième portions de surface primaire sont configurées de telle sorte que, lorsque le premier élément d'appui est déplacé et/ou déformé dans la position active, la première portion de surface primaire exerce un effort de pression sur la première capsule avant que la deuxième portion de surface primaire n'exerce un effort de pression sur la première capsule.

De façon avantageuse, les première et deuxième portions de surface primaire sont configurées pour respectivement exercer un effort de pression sur des parties arrière et avant du premier compartiment déformable.

Selon un mode de réalisation de l'invention, les première et deuxième portions de surface secondaire sont configurées de telle sorte que, lorsque le deuxième élément d'appui est déplacé et/ou déformé dans la position active, la première portion de surface secondaire exerce un effort de pression sur la deuxième capsule avant que la deuxième portion de surface secondaire n'exerce un effort de pression sur la deuxième capsule.

De façon avantageuse, les première et deuxième portions de surface secondaire sont configurées pour respectivement exercer un effort de pression sur des parties arrière et avant du deuxième compartiment déformable.

Selon un mode de réalisation de l'invention, le premier élément d'appui comporte une première surface d'actionnement opposée à la première surface d'appui, et le deuxième élément d'appui comporte une deuxième surface d'actionnement opposée à la deuxième surface d'appui, les premier et deuxième organes d'actionnement étant configurés pour exercer des efforts de pression respectivement sur les première et deuxième surfaces d'actionnement. Avantageusement, chacune des première et deuxième surfaces d'actionnement est accessible depuis l'extérieur du dispositif de réception.

Selon un mode de réalisation de l'invention, le dispositif de réception comporte au moins un élément de guidage primaire, tel qu'une rainure de guidage, configuré pour coopérer avec au moins un élément de guidage secondaire, tel qu'un doigt de guidage, prévu sur la machine de mélange, lorsque le dispositif de réception est reçu dans la machine de mélange.

Selon un mode de réalisation de l'invention, l'appareil de fabrication est configuré pour que l'introduction du dispositif de réception dans la machine de mélange entraîne mécaniquement et automatiquement l'obturation du passage de sortie.

La présente invention concerne en outre un appareil de fabrication d'un produit cosmétique, comprenant :
- une première capsule comportant un premier compartiment contenant une quantité prédéterminée d'une première formulation,
- une deuxième capsule comportant un deuxième compartiment contenant une quantité prédéterminée d'une deuxième formulation, les première et deuxième capsules étant distinctes l'une de l'autre,
- un passage de communication configuré pour relier fluidiquement les premier et deuxième compartiments des première et deuxième capsules, et
- une machine de mélange configurée pour recevoir les première et deuxième capsules reliées fluidiquement par le passage de communication, et pour mélanger les première et deuxième formulations contenues dans les premier et deuxième compartiments de manière à obtenir le produit cosmétique,
caractérisé en ce que l'appareil de fabrication comporte en outre un passage de sortie relié fluidiquement au passage de communication et pourvu d'un orifice de sortie, et en ce que l'appareil de fabrication est configuré pour automatiquement obturer le passage de sortie lorsque les première et deuxième capsules sont reçues dans la machine de mélange, et pour automatiquement libérer le passage de sortie lorsque les première et deuxième capsules sont retirées hors de la machine de mélange.

Selon un mode de réalisation de l'invention, la première capsule comporte une première partie de connexion, et la deuxième capsule comporte une deuxième partie de connexion configurée pour être connectée à la première partie de connexion, le passage de communication étant au moins en partie formé par les première et deuxième parties de connexion.

Selon un mode de réalisation de l'invention, la première capsule comporte en outre un premier passage de liaison configuré pour relier fluidiquement le premier compartiment déformable et la première partie de connexion, et la deuxième capsule comporte en outre un deuxième passage de liaison configuré pour relier fluidiquement le deuxième compartiment déformable et la deuxième partie de connexion, le passage de communication étant en outre au moins en partie formé par les premier et deuxième passages de liaison.

La présente invention concerne également un procédé de fabrication d'un produit cosmétique, comprenant les étapes suivantes :
- prévoir un appareil de fabrication selon l'invention,
- insérer manuellement les première et deuxième capsules dans la machine de mélange,
- obturer automatiquement le passage de sortie,
- mélanger les première et deuxième formulations de manière à obtenir le produit cosmétique,
- retirer manuellement les première et deuxième capsules hors de la machine de mélange, et
- libérer automatiquement le passage de sortie.

Selon un mode de mise en œuvre du procédé de fabrication, les étapes d'insertion et d'obturation sont concomitantes.

Selon un mode de mise en œuvre du procédé de fabrication, ce dernier comprend en outre les étapes suivantes :
- retirer les première et deuxième capsules hors de la machine de mélange, et
- libérer automatiquement le passage de sortie.

Selon un mode de mise en œuvre du procédé de fabrication, les étapes de retrait et libération sont concomitantes.

De toute façon l'invention sera bien comprise à l'aide de la description qui suit en référence aux dessins schématiques annexés représentant, à titre d'exemples non limitatifs, plusieurs formes d'exécution de cet appareil de fabrication.
Figure 1 est une vue en perspective d'un appareil de fabrication selon un premier mode de réalisation de l'invention.
Figure 2 est une vue de côté d'une première capsule et d'une deuxième capsule connectées l'une à l'autre et appartenant à l'appareil de fabrication de la figure 1.
Figures 3 et 4 sont respectivement des vues de dessus des première et deuxièmes capsules de la figure 2.
Figure 5 est une vue en coupe selon la ligne V-V de la figure 2.
Figure 6 est une vue partielle en coupe selon la ligne VI-VI de la figure 3.
Figure 7 est une vue en perspective d'un dispositif de réception appartenant à l'appareil de fabrication de la figure 1, montrant le dispositif de réception en position fermée.
Figure 8 est une vue en perspective du dispositif de réception de la figure 7, en position ouverte, le dispositif de réception étant représenté en position renversée par rapport à la position illustrée sur la figure 7.
Figures 9 et 10 sont des vues éclatées, en perspective, du dispositif de réception de la figure 7.
Figures 11 et 12 sont des vues en perspective du dispositif de réception de la figure 7 équipé des première et deuxième capsules, montrant des première et deuxième parties de support respectivement dans une position d'insertion et dans une position de connexion.
Figure 13 est une vue de côté du dispositif de réception de la figure 7 équipé des première et deuxième capsules.
Figure 14 est une vue à l'échelle agrandie montrant la coopération de la première capsule avec un élément de contre-appui et un élément d'obturation prévus sur le dispositif de réception.
Figures 15 et 16 sont des vues partielles de côté du dispositif de réception de la figure 7 équipé des première et deuxième capsules, montrant un premier élément d'appui respectivement dans une position inactive et une position active.
Figures 17 et 18 sont des vues en perspective respectivement des premier et deuxièmes éléments d'appui de l'appareil de fabrication.
Figure 19 est une vue de face du premier élément d'appui de l'appareil de fabrication.
Figure 20 est une vue partielle de dessus de l'appareil de fabrication de la figure 1.
Figures 21 et 22 sont des vues partielles en perspective de l'appareil de fabrication de la figure 1.
Figures 23 et 24 sont des vues schématiques de dessus d'un appareil de fabrication selon un deuxième mode de réalisation de l'invention.

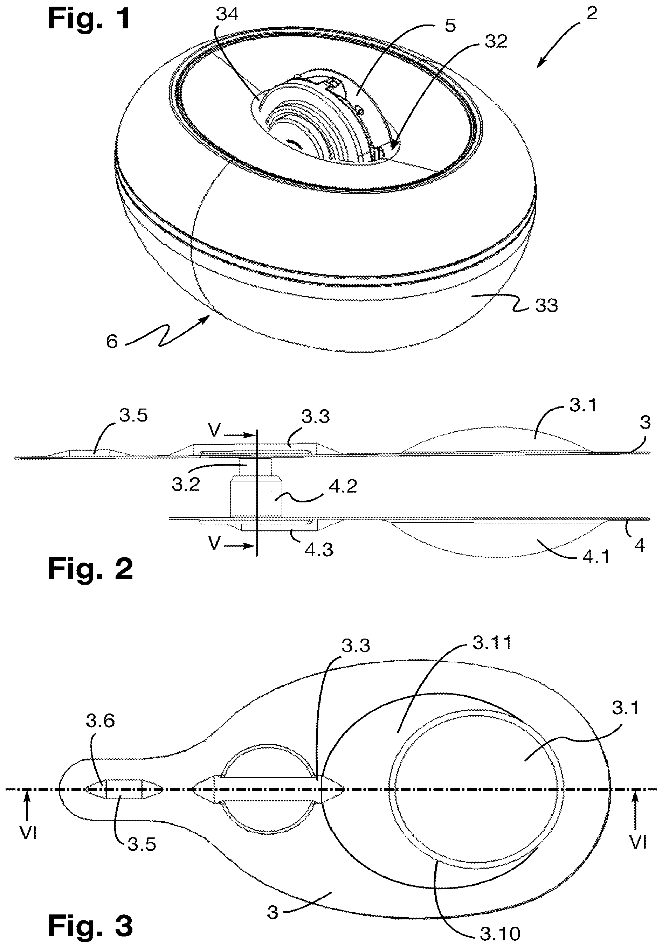

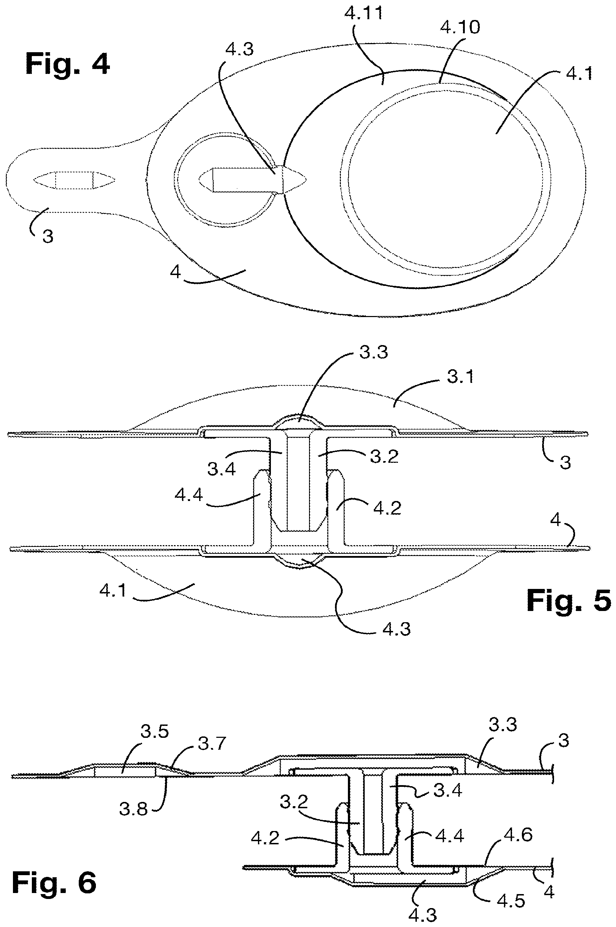

Les figures 1 à 22 représentent un appareil de fabrication 2, selon un premier mode de réalisation de l'invention, configuré pour fabriquer un produit cosmétique, qui peut par exemple être une émulsion homogénéisée, une solution homogénéisée ou encore un mélange de plusieurs phases miscibles.

L'appareil de fabrication 2 comprend en particulier des première et deuxième capsules 3, 4, également appelées dosettes ou unités de conditionnement, contenant respectivement une quantité prédéterminée d'une première formulation et une quantité prédéterminée d'une deuxième formulation, un dispositif de réception 5 configuré pour recevoir les première et deuxième capsules 3, 4, et une machine de mélange 6 configurée pour recevoir le dispositif de réception 5 équipé des première et deuxième capsules 3, 4, et pour mélanger les première et deuxième formulations contenues dans les première et deuxième capsules 3, 4 de manière à obtenir un produit cosmétique. Avantageusement, la machine de mélange 6 est configurée pour mélanger les première et deuxième formulations à l'intérieur du dispositif de réception 5, et de préférence à l'intérieur des première et deuxième capsules 3, 4.

Avantageusement, la première formulation est une première phase du produit cosmétique à fabriquer, telle qu'une phase grasse du produit cosmétique, tandis que la deuxième formulation est une deuxième phase du produit cosmétique, telle qu'une phase aqueuse du produit cosmétique. Par exemple, la phase grasse peut constituer la base du produit cosmétique à fabriquer, et la phase aqueuse peut comprendre des éléments actifs et ainsi constituer un complexe d'actifs du produit cosmétique à fabriquer.

Comme montré plus particulièrement sur les figures 2 à 6, les première et deuxième capsules 3, 4 sont distinctes l'une de l'autre, et sont configurées pour être reliées fluidiquement l'une à l'autre. En outre, chacune des première et deuxième capsules 3, 4 est avantageusement à usage unique.

La première capsule 3 comporte un premier compartiment déformable 3.1 contenant la première formulation, une première partie de connexion 3.2 et un premier passage de liaison 3.3 configuré pour relier fluidiquement le premier compartiment déformable 3.1 et la première partie de connexion 3.2. Avantageusement, le premier passage de liaison 3.3 est formé par un premier canal de liaison, et la première partie de connexion 3.2 s'étend sensiblement perpendiculairement par rapport au premier passage de liaison 3.3. La première partie de connexion 3.2 comporte plus particulièrement un embout de connexion mâle 3.4, par exemple de forme cylindrique, relié fluidiquement au premier passage de liaison 3.3.

Selon le mode de réalisation représenté sur les figures, le premier compartiment déformable 3.1 comporte une première zone périphérique frangible 3.10 présentant une ou plusieurs portions frangibles, également nommées portions d'affaiblissement, configurées pour se rompre lorsqu'une pression mécanique suffisante est exercée sur le premier compartiment déformable 3.1.

Avantageusement, la première capsule 3 comporte également une première zone tampon 3.11 s'étendant au moins en partie autour de la première zone périphérique 3.10 du premier compartiment déformable 3.1, et la ou les portions frangibles du premier compartiment déformable 3.1 sont situées de telle sorte que, lorsque la ou les portions frangibles sont rompues, le contenu du premier compartiment déformable 3.1 peut s'écouler dans la première zone tampon 3.11.

La première zone tampon 3.11 est plus particulièrement configurée pour relier fluidiquement le premier compartiment déformable 3.1 et le premier passage de liaison 3.3 lorsque la ou les portions frangibles du premier compartiment déformable 3.1 sont rompues. De façon avantageuse, avant la rupture de la ou des portions frangibles du premier compartiment déformable 3.1, les deux parois délimitant la première zone tampon 3.11 sont au contact l'une de l'autre de telle sorte que le volume de la première zone tampon 3.11 est alors sensiblement nul. Après la rupture de la ou des portions frangibles du premier compartiment déformable 3.1, l'écoulement du contenu du premier compartiment déformable 3.1 dans la première zone tampon 3.11 induit un écartement des deux parois délimitant la première zone tampon 3.11, et donc une augmentation du volume de la première zone tampon 3.11.

La première capsule 3 comporte en outre un passage de sortie 3.5, tel qu'un canal de sortie, qui est relié fluidiquement au premier passage de liaison 3.3, et qui est pourvu d'un orifice de sortie 3.6. Avantageusement, le passage de sortie 3.5 s'étend dans le prolongement du premier passage de liaison 3.3, et sensiblement parallèlement au premier passage de liaison 3.3.

Selon le mode de réalisation représenté sur les figures 1 à 22, la première capsule 3 comporte une coque thermoformée 3.7 et une feuille d'obturation 3.8 recouvrant la coque thermoformée 3.7. La coque thermoformée 3.7 et la feuille d'obturation 3.8 de la première capsule 3 délimitent avantageusement le premier compartiment déformable 3.1, le premier passage de liaison 3.3 et le passage de sortie 3.5.

La deuxième capsule 4 comporte un deuxième compartiment déformable 4.1 contenant la deuxième formulation, une deuxième partie de connexion 4.2 configurée pour être connectée à la première partie de connexion 4.1, et un deuxième passage de liaison 4.3 configuré pour relier fluidiquement le deuxième compartiment déformable 4.1 et la deuxième partie de connexion 4.2. Avantageusement, le deuxième passage de liaison 4.3 est formé par un deuxième canal de liaison, et la deuxième partie de connexion 4.2 s'étend sensiblement perpendiculairement par rapport au deuxième passage de liaison 4.3. La deuxième partie de connexion 4.2 comporte plus particulièrement un embout de connexion femelle 4.4, par exemple de forme cylindrique, relié fluidiquement au deuxième passage de liaison 4.3 et configuré pour recevoir de manière étanche l'embout de connexion mâle 3.4.

Selon le mode de réalisation représenté sur les figures, le deuxième compartiment déformable 4.1 comporte une deuxième zone périphérique frangible 4.10 présentant une ou plusieurs portions frangibles, également nommées portions d'affaiblissement, configurées pour se rompre lorsqu'une pression mécanique suffisante est exercée sur le deuxième compartiment déformable 4.1.

Avantageusement, la deuxième capsule 4 comporte également une deuxième zone tampon 4.11 s'étendant au moins en partie autour de la deuxième zone périphérique 4.10 du deuxième compartiment déformable 4.1, et la ou les portions frangibles du deuxième compartiment déformable 4.1 sont situées de telle sorte que, lorsque la ou les portions frangibles sont rompues, le contenu du deuxième compartiment déformable 4.1 peut s'écouler dans la deuxième zone tampon 4.11.

La deuxième zone tampon 4.11 est plus particulièrement configurée pour relier fluidiquement le deuxième compartiment déformable 4.1 et le deuxième passage de liaison 4.3 lorsque la ou les portions frangibles du deuxième compartiment déformable 4.1 sont rompues. De façon avantageuse, avant la rupture de la ou des portions frangibles du deuxième compartiment déformable 4.1, les deux parois délimitant la deuxième zone tampon 4.11 sont au contact l'une de l'autre de telle sorte que le volume de la deuxième zone tampon 4.11 est alors sensiblement nul.

Selon le mode de réalisation représenté sur les figures 1 à 22, la deuxième capsule 4 comporte une coque thermoformée 4.5 et une feuille d'obturation 4.6 recouvrant la coque thermoformée 4.5. La coque thermoformée 4.5 et la feuille d'obturation 4.6 de la deuxième capsule 4 délimitent avantageusement le deuxième compartiment déformable 4.1 et le deuxième passage de liaison 4.3.

De façon avantageuse et pour des raisons évoquées ci-après, chacune des première et deuxième capsules 3, 4 est configurée pour contenir la totalité ou sensiblement la totalité d'un mélange formé par la quantité prédéterminée de la première formulation et la quantité prédéterminée de la deuxième formulation.

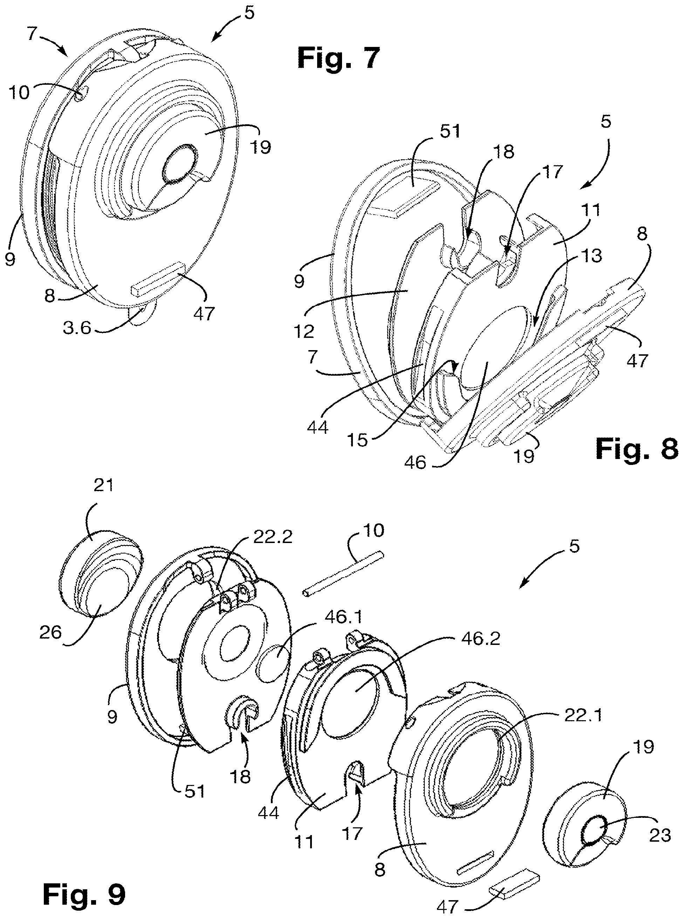

Comme montré plus particulièrement sur les figures 7 à 14, le dispositif de réception 5 est apte à occuper une position ouverte dans laquelle les première et deuxième capsules 3, 4 sont aptes à être introduites dans le dispositif de réception 5, et une position fermée dans laquelle le dispositif de réception 5 est apte à maintenir en position les première et deuxième capsules 3, 4.

Le dispositif de réception 5 comporte plus particulièrement un boîtier de réception 7 configuré pour recevoir et loger au moins en partie les première et deuxième capsules 3, 4. Le boîtier de réception 7 comporte notamment une première coque de protection 8 et une deuxième coque de protection 9 montées articulées l'une par rapport à l'autre autour d'un axe d'articulation 10 et entre une première position (voir la figure 8) correspondant à une position ouverte du dispositif de réception 5 et une deuxième position (voir la figure 7) correspondant à une position fermée du dispositif de réception 5. Les première et deuxième coques de protection 8, 9 peuvent par exemple présenter un angle d'inclinaison compris entre 50 et 90°, et par exemple d'environ 70°, lorsqu'elles sont dans la première position.

Le dispositif de réception 5 comporte en outre une première partie de support 11 et une deuxième partie de support 12 disposées dans le boîtier de réception 7. Les première et deuxième parties de support 11, 12 comportent respectivement un premier emplacement de réception 13 configuré pour recevoir la première capsule 3 et un deuxième emplacement de réception 14 configuré pour recevoir la deuxième capsule 4. Avantageusement, le premier emplacement de réception 13 comporte une première rainure de réception 15 configurée pour recevoir une portion périphérique de la première capsule 3, et le deuxième emplacement de réception 14 comporte une deuxième rainure de réception (non visible sur les figures) configurée pour recevoir une portion périphérique de la deuxième capsule 4.

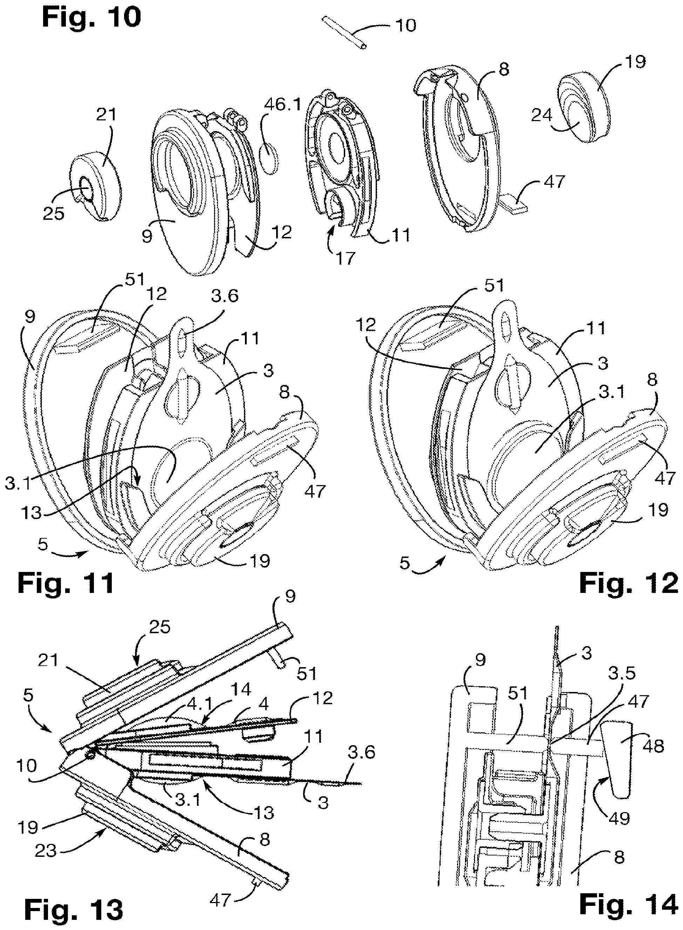

Selon le mode de réalisation représenté sur les figures 1 à 22, les première et deuxième parties de support 11, 12 sont articulées l'une par rapport à l'autre autour de l'axe d'articulation 10 et entre une position de réception (voir les figures 8 et 13) dans laquelle les première et deuxième parties de support 11, 12 sont éloignées l'une de l'autre et les première et deuxième capsules 3, 4 sont aptes à être reçues respectivement dans les premier et deuxième emplacements de réception 13, 14, et une position de connexion (voir la figure 12) dans laquelle les première et deuxième parties de support 11, 12 sont rapprochées l'une de l'autre et les première et deuxième capsules 3, 4 sont aptes à être connectées l'une à l'autre. Les première et deuxième parties de support 11, 12 peuvent par exemple présenter un angle d'inclinaison compris entre 5 et 20°, et par exemple d'environ 10°, lorsqu'elles sont dans la position de réception, et être sensiblement parallèles l'une par rapport à l'autre lorsqu'elles sont dans la position de connexion.

Avantageusement, les première et deuxième parties de support 11, 12 sont configurées pour être déplacées dans la position de connexion lorsque le dispositif de réception 5 est déplacé dans la position fermée, et donc pour relier fluidiquement les première et deuxième parties de connexion 3.2, 4.2 lorsque le dispositif de réception 5 est déplacé dans la position fermée.

La première partie de support 11 comporte en outre un premier élément de détrompage 17, tel qu'une première encoche de détrompage, configuré pour coopérer avec la première partie de connexion 3.2 de la première capsule 3, et la deuxième partie de support 12 comporte un deuxième élément de détrompage 18, tel qu'une deuxième encoche de détrompage, configuré pour coopérer avec la deuxième partie de connexion 4.2. Les premier et deuxième éléments de détrompage 17, 18 permettent d'assurer un positionnement de la première capsule 3 uniquement sur la première partie de support 11, et un positionnement de la deuxième capsule 4 uniquement sur la deuxième partie de support 12, et évitent ainsi toute erreur de positionnement des première et deuxième capsules 3, 4 dans le dispositif de réception 5.

Les première et deuxième parties de support 11, 12 sont plus particulièrement configurées de telle sorte que les première et deuxième capsules 3, 4 s'étendent sensiblement parallèlement l'une par rapport à l'autre, lorsque les première et deuxième parties de support 11, 12 sont dans la position de connexion.

Comme montré sur les figures 7 et 13, la première capsule 3 est configurée pour s'étendre en partie à l'extérieur du dispositif de réception 5 lorsqu'elle est reçue dans le dispositif de réception 5 et que ce dernier est dans la position fermée. Avantageusement, l'orifice de sortie 3.6 est configuré pour s'étendre à l'extérieur du dispositif de réception 5 lorsque la première capsule 3 est reçue dans le dispositif de réception 5 et que ce dernier est dans la position fermée.

Comme montré notamment sur les figures 9, 15 et 16, le dispositif de réception 5 comporte en outre un premier élément d'appui 19 configuré pour exercer un effort de pression sur la première capsule 3, et plus particulièrement sur le premier compartiment déformable 3.1, et un deuxième élément d'appui 21 configuré pour exercer un effort de pression sur la deuxième capsule 4, et plus particulièrement sur le deuxième compartiment déformable 4.1.

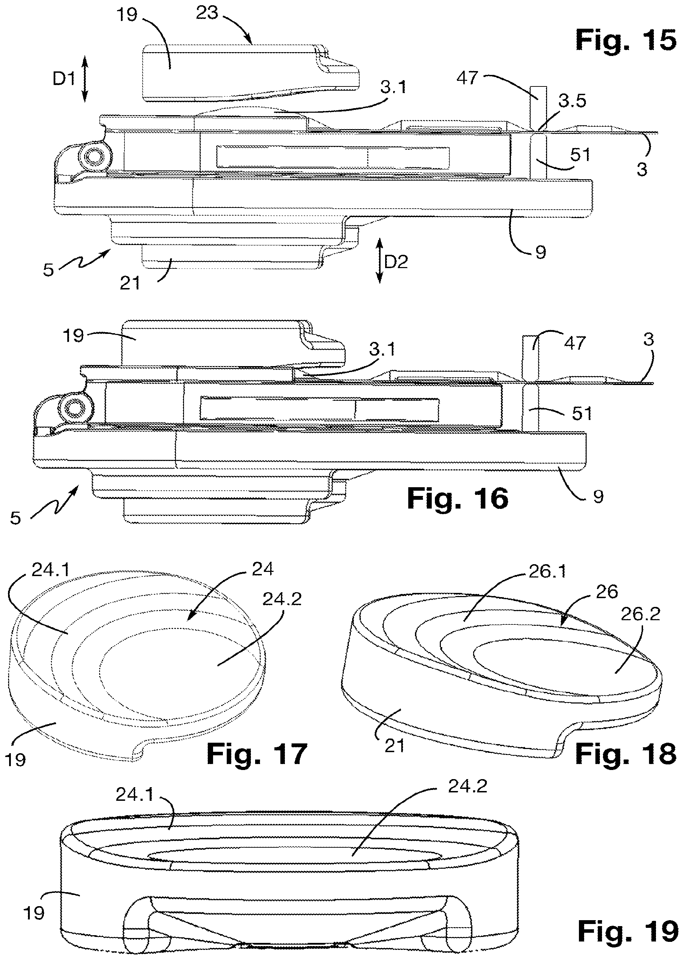

Le premier élément d'appui 19 est monté sur la première coque de protection 8 et est déplaçable entre une position inactive (voir la figure 15) et une position active (voir la figure 16) dans laquelle le premier élément d'appui 19 est apte à exercer un effort de pression sur le premier compartiment déformable 3.1. Le premier élément d'appui 19 peut par exemple être monté mobile en translation selon une première direction de déplacement D1 qui est transversale, et de préférence sensiblement orthogonale, aux plans d'extension des première et deuxième capsules 3, 4 lorsque le dispositif de réception 5 équipé des première et deuxième capsules 3, 4 est dans la position fermée.

Selon le mode de réalisation représenté sur les figures 1 à 22, le premier élément d'appui 19 s'étend à travers une première ouverture de passage 22.1 prévue sur la première coque de protection 8, et comporte une première surface d'actionnement 23 accessible depuis l'extérieur du dispositif de réception 5, et plus précisément depuis l'extérieur du boîtier de réception 7. La fonction de la première surface d'actionnement 23 sera définie ultérieurement.

Le premier élément d'appui 19 comporte en outre une première surface d'appui 24 opposée à la première surface d'actionnement 23 et s'étendant à l'intérieur du dispositif de réception 5. La première surface d'appui 24 est configurée pour coopérer avec le premier compartiment déformable 3.1, et pour guider et orienter le contenu de la première capsule 3 vers le premier passage de liaison 3.3 du premier compartiment déformable 3.1, lorsque le premier élément d'appui 19 exerce un effort de pression sur le premier compartiment déformable 3.1.

La première surface d'appui 24 comporte une première portion de surface primaire 24.1 configurée pour exercer un effort de pression sur une première portion du premier compartiment déformable 3.1, et une deuxième portion de surface primaire 24.2 configurée pour exercer un effort de pression sur une deuxième portion du premier compartiment déformable 3.1 qui est plus proche du premier passage de liaison 3.3 que la première portion du premier compartiment déformable 3.1. Selon le mode de réalisation représenté sur les figures 1 à 22, la première portion de surface primaire 24.1 est configurée pour exercer, sur la première portion du premier compartiment déformable 3.1, un effort de pression qui est orienté obliquement par rapport à la première direction de déplacement D1 et sensiblement vers le premier passage de liaison 3.3, et la deuxième portion de surface primaire 24.2 est configurée pour exercer, sur la deuxième portion du premier compartiment déformable 3.1, un effort de pression qui est orienté sensiblement parallèlement par rapport à la première direction de déplacement D1 ou qui est orienté obliquement par rapport à la première direction de déplacement et sensiblement vers le premier passage de liaison 3.3.

Avantageusement, la première portion de surface primaire 24.1 est configurée pour exercer un effort de pression notamment sur une partie arrière du premier compartiment déformable 3.1, c'est-à-dire une partie du premier compartiment déformable 3.1 qui est opposée au premier passage de liaison 3.3, et la deuxième portion de surface primaire 24.2 est configurée pour exercer un effort de pression notamment sur une partie avant du premier compartiment déformable 3.1, c'est-à-dire une partie du premier compartiment déformable 3.1 qui est tournée vers le premier passage de liaison 3.3.

Selon le mode de réalisation représenté sur les figures 1 à 22, la première portion de surface primaire 24.1 est formée par une protubérance, par exemple sensiblement arquée, prévue sur le premier élément d'appui 19, et la deuxième portion de surface primaire 24.2 est sensiblement plane, et au moins en partie entourée par la première portion de surface primaire 24.1.

Les première et deuxième portions de surface primaire 24.1, 24.2 sont plus particulièrement configurées de telle sorte que, lorsque le premier élément d'appui 19 est déplacé vers la position active, la première portion de surface primaire 24.1 exerce un effort de pression sur le premier compartiment 3.1 avant que la deuxième portion de surface primaire 24.2 n'exerce un effort de pression sur le premier compartiment 3.1.

Le deuxième élément d'appui 21 étant identique au premier élément d'appui 19, la structure et le fonctionnement du deuxième élément d'appui 21 ne sont pas décrits en détails ci-après à des fins de concision.

En particulier, le deuxième élément d'appui 21 s'étend à travers une deuxième ouverture de passage 22.2 prévue sur la deuxième coque de protection 9, et comporte une deuxième surface d'actionnement 25 accessible depuis l'extérieur du boîtier de réception 7, et une deuxième surface d'appui 26 opposée à la deuxième surface d'actionnement 25. La deuxième surface d'appui 26 est configurée pour coopérer avec le deuxième compartiment déformable 4.1, et pour guider et orienter le contenu de la deuxième capsule 4 vers le deuxième passage de liaison 4.3 du deuxième compartiment déformable 4.1, lorsque le deuxième élément d'appui 21 exerce un effort de pression sur le deuxième compartiment déformable 4.1.

Avantageusement, la deuxième surface d'appui 26 comporte également des première et deuxième portions de surface secondaire 26.1, 26.2 configurées de telle sorte que, lorsque le deuxième élément d'appui 21 est déplacé vers la position active, la première portion de surface secondaire 26.1 exerce un effort de pression sur le deuxième compartiment 4.1 avant que la deuxième portion de surface secondaire 26.2 n'exerce un effort de pression sur le deuxième compartiment 4.1.

De façon avantageuse, la première portion de surface secondaire 26.1 est configurée pour exercer un effort de pression notamment sur une partie arrière du deuxième compartiment déformable 4.1, c'est-à-dire une partie du deuxième compartiment déformable 4.1 qui est opposée au deuxième passage de liaison 4.3, et la deuxième portion de surface secondaire 26.2 est notamment configurée pour exercer un effort de pression sur une partie avant du deuxième compartiment déformable 4.1, c'est-à-dire une partie du deuxième compartiment déformable 4.1 qui est tournée vers le deuxième passage de liaison 4.3.

En outre, comme le premier élément d'appui 19, le deuxième élément d'appui 21 peut également être monté mobile en translation selon une deuxième direction de déplacement D2 qui est transversale, et de préférence sensiblement orthogonale, aux plans d'extension des première et deuxième capsules 3, 4 lorsque le dispositif de réception 5 équipé des première et deuxième capsules 3, 4 est dans la position fermée.

Chacun des premier et deuxième éléments d'appui 19, 21 peut par exemple être réalisé en un matériau au moins en partie déformable, et par exemple en silicone.

Le dispositif de réception 5 comporte en outre un élément de verrouillage (non représenté sur les figures), tel qu'un doigt de verrouillage, configuré pour verrouiller le dispositif de réception 5 dans la position fermée, et plus particulièrement pour verrouiller les première et deuxième coques de protection 8, 9 dans la deuxième position. Avantageusement, l'élément de verrouillage est mobile entre une position de verrouillage dans laquelle l'élément de verrouillage verrouille les première et deuxième coques de protection 8, 9 dans la deuxième position, et une position de déverrouillage dans laquelle l'élément de verrouillage autorise un déplacement des première et deuxième coques de protection 8, 9 dans la première position.

De façon avantageuse, le dispositif de réception 5 comporte également un élément de libération (non représenté sur les figures), tel qu'un bouton de libération, configuré pour déplacer l'élément de verrouillage dans la position de déverrouillage. Avantageusement, l'élément de libération est mobile entre une position inactive et une position d'actionnement dans laquelle l'élément de libération est apte à déplacer l'élément de verrouillage dans la position de déverrouillage. De façon avantageuse, le dispositif de réception 5 comporte un élément de sollicitation (non représenté sur les figures) configuré pour solliciter l'élément de libération dans la position inactive.

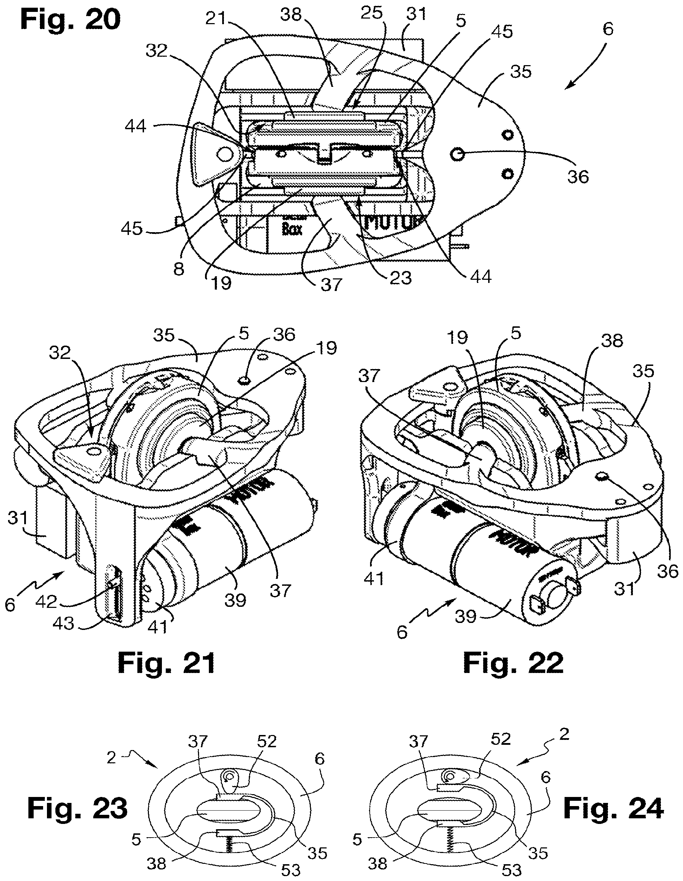

Comme montré plus particulièrement sur les figures 20 à 22, la machine de mélange 6 comporte un support 31, et un logement de réception 32 défini au moins en partie par le support 31 et configuré pour recevoir au moins en partie le dispositif de réception 5. Selon le mode de réalisation représenté sur les figures 1 à 22, la machine de mélange 6 et le dispositif de réception 5 sont configurés de telle sorte que le dispositif de réception 5 s'étend au moins en partie à l'extérieur de la machine de mélange 6, lorsque le dispositif de réception 5 est reçu dans le logement de réception 32.

La machine de mélange 6 comporte également une base 33 dans laquelle est logé le support 31, et une ouverture d'insertion 34 débouchant dans le logement de réception 32, le dispositif de réception 5 étant configuré pour être inséré dans le logement de réception 32 à travers l'ouverture d'insertion 34. Avantageusement, l'ouverture d'insertion 34 est ménagée dans une portion centrale d'une surface supérieure de la base 33, et est configurée pour être orientée vers le haut lorsque la machine de mélange 6 est disposée sur une surface de support horizontale.

La machine de mélange 6 comporte en outre une partie d'actionnement 35 montée pivotante sur le support 31 autour d'un axe de pivotement 36 sensiblement vertical lorsque la machine de mélange 6 est disposé sur une surface de support horizontale.

La partie d'actionnement 35 comporte un premier organe d'actionnement 37, tel qu'un premier doigt d'actionnement, configuré pour transmettre un effort de pression à la première capsule 3, et un deuxième organe d'actionnement 38, tel qu'un deuxième doigt d'actionnement, opposé au premier organe d'actionnement 37 et configuré pour transmettre un effort de pression à la deuxième capsule 4. Les premier et deuxième organes d'actionnement 37, 38 sont configurés pour être disposés de part et d'autre du dispositif de réception 5 lorsque ce dernier est reçu dans la machine de mélange 6, et plus précisément dans le logement de réception 32.

Les premier et deuxième organes d'actionnement 37, 38 sont plus particulièrement configurés pour exercer des efforts de pression respectivement et alternativement sur les premier et deuxièmes éléments d'appui 19, 21, de manière à transmettre des efforts de pression respectivement et alternativement sur les premier et deuxième compartiment 3.1, 4.1. En particulier, les premier et deuxième organes d'actionnement 37, 38 sont configurés pour coopérer respectivement avec les première et deuxième surfaces d'actionnement 23, 25 des premier et deuxième éléments d'appui 19, 21.

Selon le mode de réalisation représenté sur les figures 1 à 22, les premier et deuxième organes d'actionnement 37, 38 s'étendent sensiblement dans un même plan d'extension, et convergent à l'opposé de l'axe de pivotement 36.

La machine de mélange 6 comporte de plus un moteur d'entraînement 39 monté sur le support 31. Le moteur d'entraînement 39 est configuré pour faire pivoter la partie d'actionnement 35 autour de l'axe de pivotement 36 et dans une plage angulaire prédéterminée.

Selon le mode de réalisation représenté sur les figures 1 à 22, la machine de mélange 6 comporte également une roue d'entraînement 41 solidaire en rotation d'un arbre de sortie du moteur d'entraînement 39 et configurée pour être entraînée en rotation autour de son axe de roue et dans un sens de rotation par le moteur d'entraînement 39. La roue d'entraînement 41 est équipée d'un élément d'entraînement 42, tel qu'un doigt d'entraînement, qui est excentré par rapport à l'axe de roue, et qui est reçu dans une lumière de réception 43 prévue sur la partie d'actionnement 35. Avantageusement, la lumière de réception 43 est allongée et s'étend selon une direction d'extension sensiblement parallèle à l'axe de pivotement 36. Une telle configuration de la machine de mélange 6 permet d'obtenir un mouvement alternatif de la partie d'actionnement 35 en faisant tourner le moteur d'entraînement 39 toujours dans le même sens de rotation, de sorte qu'il n'est pas nécessaire de faire appel un système de commande couteux du moteur d'entraînement 39.

Selon une variante de réalisation de l'invention, la machine de mélange 6 pourrait être configurée de telle sorte qu'une rotation du moteur d'entraînement 39 dans un premier sens de rotation entraîne un pivotement de la partie d'actionnement 35 dans un premier sens de pivotement et qu'une rotation du moteur d'entraînement 39 dans un deuxième sens de rotation, opposé au premier sens de rotation, entraîne un pivotement de la partie d'actionnement 35 dans un deuxième sens de pivotement, opposé au premier sens de pivotement.

Selon le mode de réalisation représenté sur les figures 1 à 22, le dispositif de réception 6 comporte (voir la figure 20) deux éléments de guidage primaires 44, tel que des rainures de guidage, configurés pour coopérer respectivement avec des éléments de guidage secondaires 45, tel que des doigts de guidage ou des pattes de guidage, prévus sur la machine de mélange 6, lorsque le dispositif de réception 5 est reçu dans la machine de mélange 6. Les éléments de guidage primaires 44 peuvent par exemple être prévus sur la première partie de support 11, et les éléments de guidage secondaires 45 peuvent par exemple être prévus sur le support 31. La présence de tels éléments de guidage permet de faciliter le positionnement du dispositif de réception 5 dans le logement de réception 32.

L'appareil de fabrication 2 comporte en outre un élément de chauffage 46 configuré pour chauffer au moins la première capsule 3 lorsque le dispositif de réception 5 équipé des première et deuxième capsules 3, 4 est reçu dans la machine de mélange 6. L'élément de chauffage 46 peut par exemple comprendre un élément résistif chauffant 46.1 et une plaque de chauffage 46.2 adjacente à l'élément résistif chauffant 46.1.

Selon un mode de réalisation représenté sur les figures, l'élément de chauffage 46 est disposé dans le dispositif de réception 5, et est avantageusement monté sur la première partie de support 11 de manière à s'étendre entre les première et deuxième capsules 3, 4 lorsque ces dernières sont reçues dans le dispositif de réception 5. De préférence, l'élément de chauffage 46 est agencé pour s'étendre à proximité du premier compartiment déformable 3.1, et par exemple au contact du premier compartiment déformable 3.1. Cependant, selon un autre mode de réalisation de l'invention, l'élément de chauffage 46 pourrait être disposé dans la machine de mélange 6 et être configuré pour s'étendre entre les première et deuxième capsules 3, 4 lorsque le dispositif de réception 5 équipé des première et deuxième capsules 3, 4 est reçu dans la machine de mélange 6.

Selon un mode de réalisation de l'invention, la machine de mélange 6 comporte également une source d'alimentation électrique (non représentée sur les figures) configurée pour alimenter électriquement la machine de mélange 6, et notamment le moteur d'entraînement 39. La source d'alimentation électrique peut par exemple comporter au moins une batterie rechargeable.

Lorsque l'élément de chauffage 46 est disposé dans le dispositif de réception 5, la source d'alimentation électrique est avantageusement également configurée pour alimenter électriquement le dispositif de réception 5, et notamment l'élément de chauffage 46, lorsque le dispositif de réception 5 est reçu dans la machine de mélange 6. A cet effet, la machine de mélange 6 comporte un premier connecteur électrique (non représenté sur les figures), et le dispositif de réception 5 comporte un deuxième connecteur électrique (non représenté sur les figures) configuré pour être connecté au premier connecteur électrique lorsque le dispositif de réception 5 est reçu dans la machine de mélange 6, de telle sorte que la machine de mélange 6 est apte à alimenter électriquement le dispositif de réception 5.

La machine de mélange 6 comporte en outre un contrôleur, par exemple pourvu d'un microcontrôleur, configuré pour contrôler le fonctionnement du moteur d'entraînement 39 et de l'élément de chauffage 46.

L'appareil de fabrication 2 comporte en outre un élément d'obturation 47 configuré pour obturer automatiquement le passage de sortie 3.5 de la première capsule 3 lorsque le dispositif de réception 5, équipé des première et deuxième capsules 3, 4, est reçu dans la machine de mélange 6, et pour libérer automatiquement le passage de sortie 3.5 lorsque le dispositif de réception 5 équipé des première et deuxième capsules 3, 4 est retiré hors de la machine de mélange 6. Avantageusement, l'élément d'obturation 47 est configuré pour exercer une pression sur une paroi du passage de sortie 3.5 de manière à obturer ce dernier.

L'élément d'obturation 47 peut par exemple être monté mobile sur la première coque de protection 8 entre une position d'obturation (voir les figures 14 et 15) dans laquelle l'élément d'obturation 47 est apte à obturer le passage de sortie 3.5, et une position de libération (voir la figure 12) dans laquelle l'élément d'obturation 47 est apte à libérer le passage de sortie 3.5, et la machine de mélange 6 peut par exemple comporter un élément de déplacement 48 configuré pour déplacer l'élément d'obturation 47 dans la position d'obturation lorsque le dispositif de réception 5 est inséré dans la machine de mélange 6.

Selon le mode de réalisation représenté sur les figures 1 à 22, l'élément de déplacement 48 est monté fixe par rapport au support 31 et comporte une rampe de déplacement 49 (voir la figure 14) configurée pour coopérer avec l'élément d'obturation 47 et déplacer ce dernier vers la position de libération.

Avantageusement, l'appareil de fabrication 2 comporte un organe de sollicitation (non représenté sur les figures) configuré pour solliciter l'élément d'obturation 47 vers la position de libération. Ces dispositions assurent un déplacement automatique de l'élément d'obturation 47 dans la position de libération dès que le dispositif de réception 5 est retiré hors de la machine de mélange 6.

Selon le mode de réalisation représenté sur les figures 1 à 22, l'appareil de fabrication 2 comporte en outre un élément de contre-appui 51, tel qu'une nervure ou une patte de contre-appui, configuré pour prendre appui contre la première capsule 3 et pour être disposé en regard de l'élément d'obturation 47 lorsque le dispositif de réception 5 équipé des première et deuxième capsules 3, 4 est reçu dans la machine de mélange 6. Avantageusement, l'élément de contre-appui 51 est prévu sur le dispositif de réception 5, et par exemple sur la deuxième coque de protection 9. La présence d'un tel élément de contre-appui assure un pincement optimal du passage de sortie 3.5 lorsque le dispositif de réception 5 est reçu dans la machine de mélange 6.

Un procédé de fabrication d'un produit cosmétique à l'aide de l'appareil de fabrication 2 va maintenant être décrit. Un tel procédé de fabrication comprend notamment les étapes suivantes :
- prévoir l'appareil de fabrication 2,
- déplacer les première et deuxième coques de protection 8, 9 dans la première position,
- déplacer les première et deuxième parties de support 11, 12 dans la position de réception,
- insérer les première et deuxième capsules 3, 4 respectivement dans les premier et deuxième emplacements de réception 13, 14,
- déplacer les première et deuxième coques de protection 8, 9 dans la deuxième position de manière à déplacer les première et deuxième parties de support 11, 12 dans la position de connexion, et donc de manière à connecter les première et deuxième parties de connexion 3.2, 4.2,
- insérer le dispositif de réception 5 équipé des première et deuxième capsules 3, 4 dans le logement de réception 32 de la machine de mélange 6,
- déplacer automatiquement l'élément d'obturation 47 dans la position d'obturation de manière à obturer automatiquement le passage de sortie 3.5 de la première capsule 3,
- chauffer le premier compartiment déformable 3.1 et la première formulation contenue dans ce dernier, et
- mélanger les première et deuxième formulations à l'intérieur des première et deuxième capsule 3, 4 de manière à obtenir le produit cosmétique.

L'étape de mélange comporte plus particulièrement les étapes suivantes :
- faire pivoter la partie d'actionnement 35 dans le premier sens de pivotement de telle sorte que le premier organe d'actionnement 37 exerce un effort de pression sur le premier élément d'appui 19 et déplace ce dernier dans la position active ; un tel déplacement du premier élément d'appui 19 induisant une surpression au niveau de la première zone périphérique frangible 3.10 du premier compartiment déformable 3.1 et donc une rupture de la ou des portions frangibles respectives, puis un écoulement de la première formulation, contenue dans le premier compartiment déformable 3.1, jusque dans la deuxième zone tampon 4.11 via le premier passage de liaison 3.3 et le deuxième passage de liaison 4.3 ; un tel écoulement de la première formulation induisant une surpression au niveau de la deuxième zone périphérique frangible 4.10 du deuxième compartiment déformable 4.1, et donc une rupture de la ou des portions frangibles respectives et une pénétration de la première formulation dans le deuxième compartiment déformable 4.1,
- faire pivoter la partie d'actionnement 35 dans le deuxième sens de pivotement de telle sorte que le deuxième organe d'actionnement 38 exerce un effort de pression sur le deuxième élément d'appui 21, de manière à déplacer ce dernier dans la position active et à induire un écoulement du mélange des première et deuxième formulations contenues dans le deuxième compartiment déformable 4.1 jusque dans le premier compartiment déformable 3.1, et
- faire pivoter la partie d'actionnement 35 successivement dans le premier sens de pivotement et dans le deuxième sens de pivotement, et répéter une telle étape plusieurs fois, et par exemple 2 à 15 fois, avantageusement 5 à 10 fois, de manière à faire passer le mélange des première et deuxième formulation successivement dans le premier compartiment déformable 3.1 et dans le deuxième compartiment déformable 4.1, afin d'obtenir un mélange homogène des première et deuxième formulation.

Le procédé de fabrication comprend en outre les étapes suivantes :
- retirer le dispositif de réception 5 hors de la machine de mélange 6,
- déplacer automatiquement l'élément d'obturation 47 dans la position de libération de manière à libérer automatiquement le passage de sortie 3.5 de la première capsule 3,
- exercer manuellement une pression mécanique sur les premier et deuxième éléments d'appui 19, 21 de manière à expulser le produit cosmétique fabriqué hors des première et deuxième capsules 3, 4, et
- recueillir, par exemple sur les doigts d'un utilisateur, le produit cosmétique fabriqué.

Les figures 23 et 24 représentent un appareil de fabrication 2 selon un deuxième mode de réalisation de l'invention qui diffère de celui représenté sur les figures 1 à 22 notamment en ce que la partie d'actionnement 35 est montée mobile en translation par rapport au support 31 et est apte à occuper une première position dans laquelle le premier organe d'actionnement 37 est apte à exercer un effort de pression sur le premier élément d'appui 19 et une deuxième position dans laquelle le deuxième organe d'actionnement 38 est apte à exercer un effort de pression sur le deuxième élément d'appui 21, et en ce que la machine de mélange 6 comporte une came d'entraînement 52 solidaire en rotation du moteur d'entraînement 39 et configurée pour déplacer la partie d'actionnement 35 vers la première position, et un élément de sollicitation 53, tel qu'un ressort hélicoïdal, configuré pour solliciter la partie d'actionnement 35 vers la deuxième position.

Comme il va de soi, l'invention ne se limite pas aux seules formes d'exécution de cet appareil de fabrication, décrites ci-dessus à titre d'exemples, elle en embrasse au contraire toutes les variantes de réalisation selon les revendications. C'est ainsi notamment que les premier et deuxième organes d'actionnement pourraient être séparés et distincts l'un de l'autre.

## Revendications

1. Appareil de fabrication (2) d'un produit cosmétique, comprenant :
- une première capsule (3) contenant une quantité prédéterminée d'une première formulation, la première capsule (3) comportant une première partie de connexion (3.2) et un passage de sortie (3.5) pourvu d'un orifice de sortie (3.6),
- une deuxième capsule (4) contenant une quantité prédéterminée d'une deuxième formulation, et comportant une deuxième partie de connexion (4.2) configurée pour être connectée à la première partie de connexion (3.2),
- une machine de mélange (6) configurée pour recevoir les première et deuxième capsules (3, 4), et pour mélanger les première et deuxième formulations contenues dans les première et deuxième capsules (3, 4) de manière à obtenir le produit cosmétique, le passage de sortie (3.5) étant relié fluidiquement à la première partie de connexion (3.2), **caractérisé en ce que** l'appareil de fabrication (2) est configuré pour automatiquement obturer le passage de sortie (3.5) lorsque les première et deuxième capsules (3, 4) sont reçues dans la machine de mélange (6), et pour automatiquement libérer le passage de sortie (3.5) lorsque les première et deuxième capsules (3, 4) sont retirées hors de la machine de mélange (6).

2. Appareil de fabrication (2) selon la revendication 1, lequel comporte en outre un dispositif de réception (5) configuré pour recevoir les première et deuxième capsules (3, 4), la machine de mélange (6) étant configurée pour recevoir le dispositif de réception (5) équipé des première et deuxième capsules (3, 4).

3. Appareil de fabrication (2) selon la revendication 2, dans lequel le dispositif de réception (5) est apte à occuper une position ouverte dans laquelle les première et deuxième capsules (3, 4) sont aptes à être introduites dans le dispositif de réception (5), et une position fermée dans laquelle le dispositif de réception (5) est apte à maintenir en position les première et deuxième capsules (3, 4).

4. Appareil de fabrication (2) selon la revendication 3, dans lequel le dispositif de réception (5) est configuré pour relier fluidiquement les première et deuxième parties de connexion (3.2, 4.2) lorsque le dispositif de réception (5) est déplacé dans la position fermée.

5. Appareil de fabrication (2) selon l'une quelconque des revendications 1 à 4, dans lequel la première capsule (3) comporte un premier compartiment déformable (3.1) contenant la première formulation, et la deuxième capsule (4) comporte un deuxième compartiment déformable (4.1) contenant la deuxième formulation.

6. Appareil de fabrication (2) selon la revendication 5, dans lequel la première capsule (3) comporte en outre un premier passage de liaison (3.3) configuré pour relier fluidiquement le premier compartiment déformable (3.1) et la première partie de connexion (3.2), et la deuxième capsule (4) comporte en outre un deuxième passage de liaison (4.3) configuré pour relier fluidiquement le deuxième compartiment déformable (4.1) et la deuxième partie de connexion (4.2).

7. Appareil de fabrication (2) selon la revendication 6, dans lequel la première partie de connexion (3.2) s'étend sensiblement perpendiculairement par rapport au premier passage de liaison (3.3).

8. Appareil de fabrication (2) selon l'une quelconque des revendications 1 à 7, lequel comporte un élément d'obturation (47) configuré pour obturer automatiquement le passage de sortie (3.5) lorsque les première et deuxième capsules (3, 4) sont reçues dans la machine de mélange (6), et pour libérer automatiquement le passage de sortie (3.5) lorsque les première et deuxième capsules (3, 4) sont retirées hors de la machine de mélange (6).

9. Appareil de fabrication (2) selon la revendication 8, dans lequel l'élément d'obturation (47) est monté mobile entre une position d'obturation dans laquelle l'élément d'obturation (47) est apte à obturer le passage de sortie (3.5), et une position de libération dans laquelle l'élément d'obturation (47) est apte à libérer le passage de sortie (3.5).

10. Appareil de fabrication (2) selon la revendication 9, dans lequel la machine de mélange (6) comporte un élément de déplacement (48) configuré pour déplacer l'élément d'obturation (47) dans la position d'obturation lorsque les première et deuxième capsules (3, 4) sont reçues dans la machine de mélange (6).

11. Appareil de fabrication (2) selon la revendication 9 ou 10 en combinaison avec la revendication 2, lequel est configuré de telle sorte que l'introduction du dispositif de réception (5) dans la machine de mélange (6) jusqu'à une position de butée du dispositif de réception (5) entraîne mécaniquement un déplacement de l'élément d'obturation (47) dans la position d'obturation.

12. Appareil de fabrication (2) selon l'une quelconque des revendications 9 à 11 en combinaison avec la revendication 2, lequel est configuré de telle sorte que le retrait du dispositif de réception (5) hors de la machine de mélange (6) entraîne mécaniquement un déplacement de l'élément d'obturation (47) dans la position de libération.

13. Appareil de fabrication (2) selon l'une quelconque des revendications 8 à 12 en combinaison avec la revendication 2, dans lequel le dispositif de réception (5) comporte l'élément d'obturation (47).

14. Appareil de fabrication (2) selon l'une quelconque des revendications précédentes en liaison avec la revendication 2, dans lequel l'appareil de fabrication (2) est configuré pour que l'introduction du dispositif de réception (5) dans la machine de mélange (6) entraîne mécaniquement et automatiquement l'obturation du passage de sortie (3.5).

15. Procédé de fabrication d'un produit cosmétique, comprenant les étapes suivantes :
- prévoir un appareil de fabrication (2) selon l'une quelconque des revendications 1 à 14,
- insérer manuellement les première et deuxième capsules (3, 4) dans la machine de mélange (6),
- obturer automatiquement le passage de sortie (3.5),
- mélanger automatiquement les première et deuxième formulations de manière à obtenir le produit cosmétique,
- retirer manuellement les première et deuxième capsules (3, 4) hors de la machine de mélange (6), et
- libérer automatiquement le passage de sortie (3.5).

## Patentansprüche

1. Einrichtung zur Herstellung (2) eines kosmetischen Produkts, Folgendes umfassend:
- eine erste Kapsel (3), die eine vorbestimmte Menge einer ersten Formulierung enthält, wobei die erste Kapsel (3) einen ersten Verbindungsabschnitt (3.2) und einen Ausgangsdurchgang (3.5) beinhaltet, der mit einer Ausgangsöffnung (3.6) versehen ist,
- eine zweite Kapsel (4), die eine vorbestimmte Menge einer zweiten Formulierung enthält, und einen zweiten Verbindungsabschnitt (4.2), der konfiguriert ist, um mit dem ersten Verbindungsabschnitt (3.2) verbunden zu werden,
- eine Mischmaschine (6), die konfiguriert ist, um die erste und zweite Kapsel (3, 4) aufzunehmen, um die erste und zweite Formulierung, die in der ersten und zweiten Kapsel (3, 4) enthalten sind, derart zu mischen, um das kosmetisches Produkt zu erhalten,
wobei der Ausgangsdurchgang (3.5) fluidtechnisch mit dem ersten Verbindungsabschnitt (3.2) verbunden ist,
**dadurch gekennzeichnet, dass** die Einrichtung zur Herstellung (2) konfiguriert ist, um den Ausgangsdurchgang (3.5) automatisch zu verschließen, wenn die erste und zweite Kapsel (3, 4) in der Mischmaschine (6) aufgenommen werden, und um den Ausgangsdurchgang (3.5) automatisch freizugeben, wenn die erste und zweite Kapsel (3, 4) aus der Mischmaschine (6) entfernt werden.

2. Einrichtung zur Herstellung (2) nach Anspruch 1, welche weiter eine Aufnahmevorrichtung (5) beinhaltet, die konfiguriert ist, um die erste und zweite Kapsel (3, 4) aufzunehmen, wobei die Mischmaschine (6) konfiguriert ist, um die Aufnahmevorrichtung (5) aufzunehmen, die mit der ersten und zweiten Kapsel (3, 4) ausgerüstet ist.

3. Einrichtung zur Herstellung (2) nach Anspruch 2, wobei die Aufnahmevorrichtung (5) imstande ist, um eine offene Position, in der die erste und zweite Kapsel (3, 4) imstande sind, in die Aufnahmevorrichtung (5) eingebracht zu werden, und eine geschlossene Position einzunehmen, in der die Aufnahmevorrichtung (5) imstande ist, die erste und zweite Kapsel (3, 4) in Position zu halten.

4. Einrichtung zur Herstellung (2) nach Anspruch 3, wobei die Aufnahmevorrichtung (5) konfiguriert ist, um den ersten und zweiten Verbindungsabschnitt (3.2, 4.2) fluidtechnisch zu verknüpfen, wenn die Aufnahmevorrichtung (5) in die geschlossene Position bewegt wird.

5. Einrichtung zur Herstellung (2) nach einem der Ansprüche 1 bis 4, wobei die erste Kapsel (3) ein erstes verformbares Fach (3.1) beinhaltet, das die erste Formulierung enthält, und die zweite Kapsel (4) ein zweites verformbares Fach (4.1) beinhaltet, das die zweite Formulierung enthält.

6. Einrichtung zur Herstellung (2) nach Anspruch 5, wobei die erste Kapsel (3) weiter einen ersten Verknüpfungsdurchgang (3.3) beinhaltet, der konfiguriert ist, um das erste verformbare Fach (3.1) und den ersten Verbindungsabschnitt (3.2) fluidtechnisch zu verknüpfen, und die zweite Kapsel (4) weiter einen zweiten Verknüpfungsdurchgang (4.3) beinhaltet, der konfiguriert ist, um das zweite verformbare Fach (4.1) und den zweiten Verbindungsabschnitt (4.2) fluidtechnisch zu verknüpfen.

7. Einrichtung zur Herstellung (2) nach Anspruch 6, wobei sich der erste Verbindungsabschnitt (3.2) im Wesentlichen senkrecht in Bezug auf den ersten Verknüpfungsdurchgang (3.3) erstreckt.

8. Einrichtung zur Herstellung (2) nach einem der Ansprüche 1 bis 7, welche weiter ein Verschlusselement (47) beinhaltet, das konfiguriert ist, um den Ausgangsdurchgang (3.5) automatisch zu verschließen, wenn die erste und zweite Kapsel (3, 4) in der Mischmaschine (6) aufgenommen werden, und um den Ausgangsdurchgang (3.5) automatisch freizugeben, wenn die erste und zweite Kapsel (3, 4) aus der Mischmaschine (6) entfernt werden.

9. Einrichtung zur Herstellung (2) nach Anspruch 8, wobei das Verschlusselement (47) beweglich zwischen einer Verschlussposition, in der das Verschlusselement (47) imstande ist, den Ausgangsdurchgang (3.5) zu verschließen, und einer Freigabeposition, in der das Verschlusselement (47) imstande ist, den Ausgangsdurchgang (3.5) freizugeben, montiert ist.

10. Einrichtung zur Herstellung (2) nach Anspruch 9, wobei die Mischmaschine (6) ein Verschiebeelement (48) beinhaltet, das konfiguriert ist, um das Verschlusselement (47) in die Verschlussposition zu verschieben, wenn die erste und zweite Kapsel (3, 4) in der Mischmaschine (6) aufgenommen werden.

11. Einrichtung zur Herstellung (2) nach Anspruch 9 oder 10, in Verbindung mit Anspruch 2, welche derart konfiguriert ist, dass das Einbringen der Aufnahmevorrichtung (5) in die Mischmaschine (6) bis zu einer Anschlagposition der Aufnahmevorrichtung (5) mechanisch eine Verschiebung des Verschlusselements (47) in die Verschlussposition antreibt.

12. Einrichtung zur Herstellung (2) nach einem der Ansprüche 9 bis 11, in Verbindung mit Anspruch 2, welche derart konfiguriert ist, dass die Entnahme der Aufnahmevorrichtung (5) aus der Mischmaschine (6) heraus mechanisch eine Verschiebung des Verschlusselements (47) in die Freigabeposition antreibt.

13. Einrichtung zur Herstellung (2) nach einem der Ansprüche 8 bis 12, in Verbindung mit Anspruch 2, wobei die Aufnahmevorrichtung (5) das Verschlusselement (47) beinhaltet.

14. Einrichtung zur Herstellung (2) nach einem der vorstehenden Ansprüche, in Verbindung mit Anspruch 2, wobei die Einrichtung zur Herstellung (2) konfiguriert ist, damit das Einführen der Aufnahmevorrichtung (5) in die Mischmaschine (6) mechanisch und automatisch den Verschluss des Ausgangsdurchgangs (3.5) antreibt.

15. Verfahren zur Herstellung eines kosmetischen Produkts, die folgenden Schritte umfassend:
- Vorsehen einer Einrichtung zur Herstellung (2) nach einem der Ansprüche 1 bis 14,
- Einführen von Hand der ersten und zweiten Kapsel (3, 4) in die Mischmaschine (6),
- automatisches Verschließen des Ausgangsdurchgangs (3.5),
- automatisches Mischen der ersten und zweiten Formulierung derart, um das kosmetische Produkt zu erhalten,
- manuelles Entnehmen der ersten und zweiten Kapsel (3, 4) aus der Mischmaschine (6), und
- automatisches Freigeben des Ausgangsdurchgangs (3.5).

## Claims

1. An apparatus (2) for manufacturing a cosmetic product, comprising:
- a first capsule (3) containing a predetermined amount of a first formulation, the first capsule (3) including a first connection portion (3.2) and an outlet passage (3.5) provided with an outlet orifice (3.6),
- a second capsule (4) containing a predetermined amount of a second formulation, and including a second connection portion (4.2) configured to be connected to the first connection portion (3.2),
- a mixing machine (6) configured to receive the first and second capsules (3, 4), and to mix the first and second formulations contained in the first and second capsules (3, 4) so as to obtain the cosmetic product,
the outlet passage (3.5) being fluidly connected to the first connection portion (3.2), **characterized in that** the manufacturing apparatus (2) is configured to automatically close the outlet passage (3.5) when the first and second capsules (3, 4) are received in the mixing machine (6), and to automatically clear the outlet passage (3.5) when the first and second capsules (3, 4) are removed out of the mixing machine (6).

2. The manufacturing apparatus (2) according to claim 1, which further includes a receiving device (5) configured to receive the first and second capsules (3, 4), the mixing machine (6) being configured to receive the receiving device (5) equipped with the first and second capsules (3, 4).

3. The manufacturing apparatus (2) according to claim 2, wherein the receiving device (5) is capable of occupying an open position in which the first and second capsules (3, 4) can be introduced into the receiving device (5), and a closed position in which the receiving device (5) can hold the first and second capsules (3, 4) in position.

4. The manufacturing apparatus (2) according to claim 3, wherein the receiving device (5) is configured to fluidly connect the first and second connection portions (3.2, 4.2) when the receiving device (5) is displaced into the closed position.

5. The manufacturing apparatus (2) according to any one of claims 1 to 4, wherein the first capsule (3) includes a first deformable compartment (3.1) containing the first formulation, and the second capsule (4) includes a second deformable compartment (4.1) containing the second formulation.

6. The manufacturing apparatus (2) according to claim 5, wherein the first capsule (3) further includes a first connecting passage (3.3) configured to fluidly connect the first deformable compartment (3.1) and the first connection portion (3.2), and the second capsule (4) further includes a second connecting passage (4.3) configured to fluidly connect the second deformable compartment (4.1) and the second connection portion (4.2).

7. The manufacturing apparatus (2) according to claim 6, wherein the first connection portion (3.2) extends substantially perpendicular with respect to the first connecting passage (3.3).

8. The manufacturing apparatus (2) according to any one of claims 1 to 7, which includes a closure element (47) configured to automatically close the outlet passage (3.5) when the first and second capsules (3, 4) are received in the mixing machine (6), and to automatically clear the outlet passage (3.5) when the first and second capsules (3, 4) are removed out of the mixing machine (6).

9. The manufacturing apparatus (2) according to claim 8, wherein the closure element (47) is movably mounted between a closing position in which the closure element (47) is capable of closing the outlet passage (3.5), and a clearance position in which the closure element (47) is capable of clearing the outlet passage (3.5).

10. The manufacturing apparatus (2) according to claim 9, wherein the mixing machine (6) includes a displacement element (48) configured to displace the closure element (47) into the closing position when the first and second capsules (3, 4) are received in the mixing machine (6).

11. The manufacturing apparatus (2) according to claim 9 or 10 in combination with claim 2, which is configured such that the introduction of the receiving device (5) into the mixing machine (6) up to an abutment position of the receiving device (5) mechanically causes a displacement of the closure element (47) into the closing position.

12. The manufacturing apparatus (2) according to any one of claims 9 to 11 in combination with claim 2, which is configured such that the removal of the receiving device (5) out of the mixing machine (6) mechanically causes a displacement of the closure element (47) into the clearance position.

13. The manufacturing apparatus (2) according to any one of claims 8 to 12 in combination with claim 2, wherein the receiving device (5) includes the closure element (47).

14. The manufacturing apparatus (2) according to any one of the preceding claims in connection with claim 2, wherein the manufacturing apparatus (2) is configured such that the introduction of the receiving device (5) into the mixing machine (6) mechanically and automatically causes the closing of the outlet passage (3.5).

15. A method for manufacturing a cosmetic product, comprising the following steps of:
- providing a manufacturing apparatus (2) according to any one of claims 1 to 14,
- manually inserting the first and second capsules (3, 4) into the mixing machine (6),
- automatically closing the outlet passage (3.5),
- automatically mixing the first and second formulations so as to obtain the cosmetic product,
- manually removing the first and second capsules (3, 4) out of the mixing machine (6), and
- automatically clearing the outlet passage (3.5).
